# EUROPEAN PATENT SPECIFICATION

(11) **EP 0 496 074 B1**
(45) Date of publication and mention of the grant of the patent: **20.11.1996**
(21) Application number: 91121419.5
(22) Date of filing: 13.12.1991
(51) Int. Cl.: A61K 51/00, A61K 47/48, A61K 49/00

(54) **Biotinylated monoclonal antibodies, avidin and biotin for diagnosis and therapy**
Biotinylierte monoklonale Antikörper, Avidin und Biotin für die Diagnose und Therapie
Anticorps monoclonaux biotinylés, avidin et biotin pour la diagnose et la thérapie

(30) Priority: 21.12.1990 IT 6805390
(43) Date of publication of application: 29.07.1992
(73) Proprietor: MINISTERO DELL' UNIVERSITA' E DELLA RICERCA SCIENTIFICA E TECNOLOGICA, 00144 Roma (IT)
(72) Inventor: Paganelli, Giovanni, I-47023 Cesena (Forl ) (IT); Siccardi, Antonio, I-20124 Milano (IT); Malcovati, Massimo, I-27100 Pavia (IT); Scassellati, Gianalfredo, I-10128 Torino (IT); Fazio, Ferruccio, I-20129 Milano (IT)
(74) Representative: Rambelli, Paolo

(56) References cited:
- EP-A- 0 251 494
- WO-A-87/02893
- THE JOURNAL OF NUCLEAR MEDICINE, vol. 28, no. 8, August 1987, pages 1294-1302, New York, US; D.J. HNATOWICH et al.: "Investigations of avidin and biotin for imaging applications"
- CHEMICAL ABSTRACTS, vol. 110, no. 18, abstract no. 160325c, Columbus, Ohio, US; V.S. TRUBETSKOI et al.: "Immunoliposome complexes: preparation, characterization and directed delivery to the human endothelial cell culture"

## Description

The present invention relates to a preparation for the diagnosis and treatment of tumours and to its use for these purposes. More particularly, the invention relates to a combination or kit including reagents for sequential administration in order to fix selectively radioactive isotopes, cytotoxic drugs or paramagnetic agents to tumour cells in a mammal.

It is known that murine monoclonal antibodies specific to tumour-associated antigens expressed by human tumours have been used, after radiolabelling with suitable radioisotopes, for diagnostic purposes (radioimmunoscintigraphy) or for therapeutic purposes (radioimmunotherapy).

The diagnostic technique is based on the assumption that the aforesaid antibodies can localise on the tumour cells with sufficient selectivity to enable them to be visualised by a gamma camera. It has been found, however, that some of the antibodies are localised non-specifically, mainly in the liver, the spleen and the bone marrow. This disadvantage limits the usefulness of this diagnostic procedure because the results can only be interpreted correctly by operators who are very familiar with it, since complex technique are needed to differentiate between specific and non-specific localisations.

The use of radiolabelled monoclonal antibodies for therapeutic purposes has also been proposed but it has been found that it is not possible, in practice, to deliver a high enough dose of radioactivity (about 5000 rads) to the tumour to destroy it selectively, except when intraperitoneal or intrapleural tumours are treated by intracavitary in loco administration. When the radioactive monoclonal antibodies are administered intravenously, however, the maximum dose of radioactivity which can be delivered to the tumour without causing intolerable damage to the whole body system, is considerably less.

Within the scope of the present invention, it has been found that, when monoclonal antibodies which recognise a specific tumour-associated human antigen are first conjugated with biotin, then labelled and then administered to man by systemic or intracavitary way, their fate is similar to that of the corresponding non-biotinylated radioactive monoclonal antibodies. At any rate, they are localised mainly on the tumour cells and, to a lesser extent, at non-specific sites; they then persist on the surfaces of the tumour cells for many days whilst, at the non-specific localisations, they are internalised and catabolised by the cells within about 24-48 hours. At an appropriate time after administration, therefore, the only extracellular biotins are those anchored to the tumour cells, whilst all the biotin naturally present in the human body (vitamin H) and the exogenous biotin conjugated with that fraction of the monoclonal antibodies which, after administration, reached non-specific sites are located within cells and hence are no longer directly accessible to extracellular fluids.

The fact that the monoclonal antibody which is internalised at the sites of non-specific localisation is biotinylated but non radioactive has the advantage of preventing the radiation damage which is normally caused when radiolabelled antibodies are internalised after in vivo administration.

A further observation upon which the present invention is based is that, although avidin is a heterologous protein, it does not give rise to toxicity or immediate hypersensitivity in man and does not show non-specific localisations.

A subject of the present invention, therefore, is a kit of reagents comprising measured amounts of reagents in a form suitable for sequential intravenous administration to a mammal suffering or suspected to be suffering from solid tumours, for the diagnosis and/or treatment of such disorders, characterized in that it includes:
a) a biotinylated monoclonal antibody (or a fragment thereof) specific to a tumour-associated antigen and able to persist on the surface of the tumour for a period of not less than 48 hours,
b) a protein of the avidin type, which can bind specifically to biotin,
c) biotin or a derivative thereof, optionally conjugated or intended to be conjugated with an agent selected from radioisotopes, paramagnetic agents and cytotoxic agents, and
d) a biotinylated agent capable of remaining in the plasma for a certain period of time and capable of binding and removing circulating avidin not fixed to the tumour cells, such an agent being selected from a group of serum proteins like biotinylated albumin, biotinylated transferrin and biotinylated IgG.

The sequential use of these reagents, which enables a radioactive, cytotoxic or paramagnetic agent to be fixed selectively to tumour cells, includes the steps of:
i) administering a measured quantity of the component a) intravenously,
ii) administering a measured quantity of component b) intravenously after a suitable period of time following the administration of the component a), the period of time being long enough for the biotinylated monoclonal antibodies which are not fixed to the tumour as a tumour-associated antigen/biotinylate antibody complex to be catabolised, and
iii) administering a measured quantity of the component c) intravenously, at least 24 hours (preferably 48 hours) after the administration of the component b).

In the preferred embodiment, the administration of the component c) is preceded, by a few minutes, (from 3 to 15 minutes), by injection of a quantity of the component d) substantially equimolar to the quantity of circulating avidin, which is determined beforehand by known methods.

Suitable monoclonal antibodies usable within the scope of the present invention are those which remain on the external surfaces of tumour cells for a sufficient period of time (4-20 days) and anyway for at least 48 hours. This ability to persist on the external surfaces of the tumour cells has to be determined beforehand for each antibody by tests on animal models and tests carried out by known techniques on bioptic or autoptic samples and then subsequently confirmed on bioptic samples resulting from clinical investigations in man.

Examples of suitable monoclonal antibodies are the following murine monoclonal antibodies available from Sorin Biomedica, Saluggia (VC), Italy:
- F023C5 (directed towards CEA antigen which is expressed by carcinomas of various histotypes),
- B 72.3 (directed towards TAG-72 antigen which is expressed by epithelial carcinomas),
- 255.28S (directed towards HMW-MAA antigen which is expressed by malignant melanoma).

In the known diagnostic use (immunoscintigraphy), these antibodies are usually labelled, by known techniques, with radioisotopes emitting solely, or mainly, gamma radiation.

Examples of such radioisotopes are Indium-111, Technetium-99m, Iodine-123 and Iodine-131. The latter, which also emits beta radiation, is also used for therapeutic purposes. However, as already indicated above, when administered intravenously, these radiolabelled monoclonal antibodies cannot concentrate the radiation dose necessary to destroy the tumour (at least 5000 rads) without at the same time causing systemic damage to the patient as a result of unwanted radiation to normal tissues and cells.

In the diagnostic or therapeutic use according to the present invention, the monoclonal antibodies specific to tumour-associated human antigens which can remain on the external surfaces of the tumour cells for at least 48 hours are not administered in the form of radiolabelled antibodies. They are treated, instead, with reactive biotin derivatives (such as, for example, esters) prepared according to known techniques so as to form a compound in which biotin and the monoclonal antibody are conjugated by a covalent bond. For this purpose, one of the various commercial compounds indicated as suitable for biotinylating proteins, such as, for example, ENZOTIN^{(R)} from Enzo Biochemicals, N.Y., U.S.A., or BIOTIN X NHS from Società Prodotti Antibiotici of Milan, may conveniently be used.

The solution of the biotinylated monoclonal antibody thus obtained is dialysed against a sterile physiological solution and then sterilised by filtration through suitable cellulose filters with pores of 0.22 microns. After its sterility, apirogenicity and all the characteristics required for an injectable solution have been checked, the solution thus obtained is administered to the patient.

The biotinylated monoclonal antibodies are stable and are therefore suitable for commercial preparations in large batches according to known techniques. If reagents are to be supplied in a commercial kit form, bottles containing the monoclonal antibody either in the form of freeze-dried powder or as a sterile pyrogen-free solution ready for use are provided.

The quantity of biotinylated monoclonal antibodies injected will vary according to parameters known in the art, such as, for example, the body weight of the patient, his physiopathological conditions and the purpose (diagnostic or therapeutic) to be achieved. Usually, however, it will vary between 0.007 and 0.14 mg/kg and, in particular, 0.007-0.028 mg/kg for diagnostic use and 0.028-0.14 for therapeutic use. Any overdose has contraindications for the patient and does not, therefore, comprise the subsequent stages since any excess of the biotinylated antibody will be eliminated, according to the present invention, by the subsequent administration of avidin. The necessary time must elapse between the administration of the biotinylated monoclonal antibody and the administration of the avidin for the biotinylated monoclonal antibodies which are localised at non-specific sites to be internalised and catabolised by the non-tumorous cells to which they are bound. This time will be at least 24 hours, preferably 48 hours.

The general term avidin indicates a group of proteins which are characterised, from a functional point of view, by the property that they bind biotin (formerly called vitamin H) with a very high affinity and specificity. They are produced by various higher organisms (birds, amphibians, etc.), wherein they are present in the eggs, or by a Streptomyces (Streptomyces avidinii). The former group of proteins are strongly basic glyco-proteins known as avidin in the strict sense; the latter is not a glycoprotein, has an isoelectric point near neutrality, and is known by the term of streptavidin.

These proteins are commercially available either in the native form or modified as a result of partial proteolysis (like the so-called core streptavidin), partial deglycosylation or chemical modification (acetylavidin, succinylavidin, etc.).

All these proteins are their derivatives, although not explicitly mentioned, will be covered by the term avidin in the present description and in the claims.

Advantageously, the injection with avidin is carried out in two successive steps: 1/5 of the dose added to 3 ml of physiological solution injected rapidly in a bolus to favour the clearance of the circulating biotinylated antibodies from the plasma, and the remaining 4/5 in 100 ml of physiological solution administered slowly (over a period of 15-30 minutes) after 5-15 minutes from the first injection. Advantageously, the kit thus includes two separate measured quantities of avidin in the proportions indicted above.

The total dose of avidin is intended to represent a molar excess of at least 10 times and at most 100 times the quantity of antibodies injected. The dose is generally between 0.07 and 1.4 mg/kg and, in particular, 0.07-0.14 mg/kg for diagnostic use and 0.7-1.4 mg/kg for therapeutic use.

The kit according to the present invention provides for the administration of avidin which is not radiolabelled (referred to below as "cold" avidin) for two distinct purposes which are both advantageous for diagnostic and/or therapeutic purposes: 1) the avidin eliminates the circulating biotinylated antibodies from the circulating plasma and concentrates them in organ such as the liver and the spleen where they are rapidly catabolised; 2) avidin enables a large quantity of a radioactive, cytotoxic or paramagnetic agent to be concentrated on the surface of the tumour as a result of the subsequent administration of biotin, with a less harmful effect on the entire organism. In fact, given that avidin can bind up to 4 units of biotin, the tumour-associated antigen/biotinylated monoclonal antibody/avidin complex will have a terminal avidin unit which can capture and fix up to 3 molecules of the biotin derivative c) mentioned above.

A preferred and characterising aspect of the present invention is the fact that the administration of a radioactive, cytotoxic or paramagnetic derivative of biotin is preceded by a few minutes, by the administration of a biotinylated agent capable of remaining in the plasma for a certain period of time and capable of binding and removing the circulating avidin not fixed to the tumour cells, such as, for example, biotinylated human albumin or an equivalent agent such a biotinylated transferrin or biotinylated immunoglobulin IgG.

The quantity of biotinylated albumin administered will be equimolar to the quantity of avidin circulating at the time of the injection. The avidin circulating is measured according to methods known to an expert in the art. This administration has the function of leading to the formation of circulating avidin-biotinylated albumin complexes, thus saturating the biotin-binding sites still present in the blood. These complexes are then captured by the liver and the spleen so that their level in the circulation is reduced. The formation of circulating complexes of avidin with radiolabelled biotin or biotin conjugated to cytotoxic or paramagnetic agents is thus reduced to minimum values thus significantly reducing the background due to these complexes circulating in the blood, particularly during the immunoscintigraphic or NMR determination.

The biotin or its derivative c) is administered after a period of at least 24 hours, and preferably 48 hours, in doses of from 2.8 to 70 µg/kg and, in particular, within the range 2.8-7 µg/kg for diagnostic use and 7-70 µg/kg for therapeutic use.

Preferably, radioisotopes selected from group a) and b) below and intended for diagnostic use and therapeutic use, respectively, are used for the preparation of the radiolabelled compound: a) Fe-52, Mn-52m, Co-55, Cu-64, Ga-67, Ga-68, Tc-99m, In-111, I-123, I-125, I-131; b) P-32, Sc-47, Cu-67, Y-90, Pd-109, Ag-111, I-131, Pm-149, Re-186, Re-188, At-211, Pb-212, Bi-212.

Within the scope of the invention, the biotin and/or derivatives thereof may also be conjugated with paramagnetic agents, such as gadolinium, iron, manganese and the like, for use in the nuclear magnetic resonance method.

Moreover, biotin and derivatives thereof may be conjugated with non-radioactive cytotoxic substances, such as ricin, Adriamycin, cis-platinum and similar chemotherapeutic agents, which are directly conjugated with the biotin or contained in biotinylated liposomes.

In the selection of a suitable radioisotope, account will be taken of known criteria such as the type of radiation emitted by the radionuclide and the purpose (diagnostic or therapeutic) to be achieved by the administration of the radioactive compound. The biotin or derivative thereof will be radiolabelled according to known techniques which produce a stable compound.

The same considerations apply to the conjugation with cytotoxic or paramagnetic agents.

The main characteristic of the radioactive biotin derivative is its low molecular weight which results in a biological half-life shorter than that of a radiolabelled antibody and which thus enables large doses of radioactivity to be administered with relatively low systemic radiation doses.

Another characteristic of the present invention is the use of "cold" avidin and "hot" biotin or a paramagnetic or cytotoxic derivative of biotin in order to fix selectively suitable radioisotopes or other agents to tumour cells in man.

Another characteristic of the present inventions is constituted by the use of the avidin-biotin system described above for detecting tumorous lesions during surgical operations for the resection of the tumour. This detection may be carried out with the use of a suitable radioactivity detection probe and suitable specific counting instrumentation, such as that supplied by Neoprobe Inc., Columbus, Ohio, U.S.A. The method could provide for the administration of cold biotinylated antibodies followed by injection of avidin and biotinylated albumin in the quantities and at the intervals described above and by the administration of biotin radiolabelled with I-125, I-123 or Tc-99m between 2 and 24 hours before the operation.

Suitable kits containing measured quantities of the components a), b), c) and, preferably d), which constitute a further subject of the present invention, may be produced commercially to enable the avidin-biotin system described to be used routinely in vivo.

A kit according to the invention typically includes:
- a bottle (or vial) containing from 0.5 to 10 mg of at least one biotinylated monoclonal antibody a),
- a bottle containing from 5 to 100 mg of avidin b) or two bottles containing 1/5 and 4/5 of the total dose, respectively,
- a bottle containing from 0.1 to 5 mg of biotin or a derivative thereof c), and
- preferably, a bottle of biotinylated albumin d) or an equivalent agent as described above, in a quantity of from 4 to 20 mg of injectable solution.

The components a), b), c) and d) may be in the form of a sterile and pyrogen-free aqueous solutions having the characteristics required for injectable products or, alternatively, in the form of sterile and pyrogen-free freeze-dried powders. In this case, the kit also includes one or more bottles containing 10-100 ml of suitable sterile and pyrogen-free injectable solutions, which may even be of different kinds but are such as to enable the preparations supplied as freeze-dried powders to be reconstituted.

## Claims

1. A kit of reagents comprising measured amounts of reagents in a form suitable for sequential intravenous administration to a mammal suffering or suspected to be suffering from solid tumours, for the diagnosis and/or treatment of such disorders, characterised in that it includes:
a) a biotinylated monoclonal antibody (or a fragment thereof) specific to a tumour-associated antigen and able to persist on the surface of the tumour for a period of not less than 48 hours,
b) a protein of the avidin type, which can bind specifically to biotin,
c) biotin or a derivative thereof, conjugated or intended to be conjugated with an agent selected from radioisotopes, paramagnetic agents and cytotoxic agents, and
d) a biotinylated agent capable of remaining in the plasma for a certain period of time and capable of binding and removing circulating avidin not fixed to the tumour cells, such an agent being selected from a group of serum proteins like biotinylated albumin, biotinylated transferrin and biotinylated IgG.

2. A kit according to claim 1, wherein the total amount of component b) is from 5 to 100 mg and is provided in a form suitable for two successive steps of intravenous administration.

3. A kit according to claim 1 or 2, in which the measured amount of component a) is between 0.5 and 10 mg.

4. A kit according to any of the preceding claims, in which the measured amount of component b) constitutes a molar excess of from 10 to 100 times the measured amount of component a).

5. A kit according to any of the preceding claims, in which component c) is radiolabelled with a radioisotope selected from one of the following groups: a) Fe-52; Mn-52m, Co-55, Cu-64, Ga-67, Ga-68, Tc-99m, In-111, I-123, I-125, I-131; b) P-32, Sc-47, Cu-67, Y-90, Pd-109, Ag-111, I-131, Pm-149, Re-186, Re-188, At-211, Pb-212, Bi-212.

6. A kit according to any of claim 1 to 4, in which component c) is conjugated with a paramagnetic agent selected from gadolinium, iron and manganese.

7. A kit according to any of claims 1 to 4, in which component c) is conjugated with a cytotoxic agent selected from ricin, Adriamycin or cis-platinum.

8. A kit according to any of claims 1 to 4, in which component c) is constituted by biotinylated liposomes including a chemotherapeutic agent.

9. A kit according to any of claim 1 or 3-8, in which the measured amount of component b) is from 0.1 to 5 mg.

10. A kit according to claim 1 or 2, in which component b) is provided in two separate dosage forms:
i) 1/5 of the total dose in 3 ml of physiological solution; and
ii) 4/5 of the dose in 100 ml of physiological solution.

## Patentansprüche

1. Ein Satz von Nachweismitteln, der abgemessene Mengen von Reagenten in einer geeigneten Form für schrittweise intravenöse Anwendung an einem Säugetier, welches an festen Tumoren leidet oder bei dem dies vermutet wird, zur Diagnose und/oder Behandlung solcher Erkrankungen aufweist, dadurch gekennzeichnet, daß er
a) einen biotinylierten monoklonalen Antikörper (oder ein Fragment davon), der zu einem mit dem Tumor assoziierten Antigen spezifisch ist und der in der Lage ist, auf der Oberfläche des Tumors für eine Zeitspanne von nicht weniger als 48 Stunden zu bestehen,
b) ein Protein des Typs Avidin, welches sich spezifisch an Biotin binden kann,
c) Biotin oder ein Derivat davon, gekoppelt mit oder dazu vorgesehen, mit einem aus Radioisotopen, paramagnetischen Mitteln und zytotoxischen Mitteln ausgewählten Agens gekoppelt zu werden, und
d) ein biotinyliertes Agens, welches in der Lage ist, für eine gewisse Zeitspanne im Plasma zu verbleiben und welches in der Lage ist, zirkulierendes, nicht an die Tumorzellen gebundenes Avidin zu binden und zu entfernen, wobei ein solches Agens aus einer Gruppe von Serumproteinen wie biotinyliertem Albumin, biotinyliertem Transferrin und biotinyliertem IgG ausgewählt wird,
enthält.

2. Ein Satz gemäß Anspruch 1, wobei die Gesamtmenge der Komponente b) zwischen 5 und 100 mg liegt und in einer für zwei aufeinanderfolgende Schritte intravenöser Anwendung geeigneten Form vorliegt.

3. Ein Satz gemäß Anspruch 1 oder 2, wobei die gemessene Menge der Komponente a) zwischen 0,5 und 10 mg liegt.

4. Ein Satz gemäß irgendeinem der vorhergehenden Ansprüche, wobei die gemessene Menge der Komponente b) einen molaren Überschuß von 10 bis 100 Mal der gemessenen Menge von Komponente a) darstellt.

5. Ein Satz gemäß einem der vorhergehenden Ansprüche, wobei die Komponente c) mit einem aus einer der folgenden Gruppen ausgewählten Radioisotop radioaktiv markiert iSt: a) Fe-52; Mn-52m, Co-55, Cu-64, Ga-67, Ga-68, Tc-99m, In-111, I-123, I-125, I-131; b) P-32, Sc-47, Cu-67, Y-90, Pd-109, Ag-111, I-131, Pm-149, Re-186, Re-188, At-211, Pb-212, Bi-212.

6. Ein Satz gemäß einem der Ansprüche 1 bis 4, wobei die Komponente c) mit einem aus Gadolinium, Eisen und Mangan ausgewählten paramagnetischen Agens gekoppelt ist.

7. Ein Satz gemäß einem der Ansprüche 1 bis 4, wobei die Komponente c) mit einem aus Rizin, Adriamyzin oder cis-Platin ausgewählten zytotoxischen Agens gekoppelt ist.

8. Ein Satz gemäß einem der Ansprüche 1 bis 4, wobei die Komponente c) aus biotinylierten Liposomen einschließlich eines chemotherapeutischen Agens besteht.

9. Ein Satz gemäß einem der Ansprüche 1 oder 3-8, wobei die gemessene Menge von Komponente b) zwischen 0,1 und 5 mg liegt.

10. Ein Satz gemäß Anspruch 1 oder 2, wobei die Komponente b) in zwei verschiedenen Dosierungsformen vorgesehen ist:
i) 1/5 der gesamten Dosis in 3 ml physiologischer Lösung; und
ii) 4/5 der Dosis in 100 ml physiologischer Lösung.

## Revendications

1. Ensemble de réactifs comprenant des quantités mesurées de réactifs sous une forme appropriée pour être administrés par voie intraveineuse, de manière séquentielle, à un mammifère souffrant de ou suspecté de présenter des tumeurs solides, pour le diagnostic et/ou le traitement de ce type d'affection,, caractérisé en ce qu'il comprend:
a) un anticorps monoclonal biotinylé (ou un fragment de dernier) spécifique d'un antigène associé à une tumeur et capable de persister à la surface de la tumeur pendant une période d'au moins 48 heures,
b) une protéine du type avidine, qui peut se lier spécifiquement à la biotine,
c) de la biotine, ou un dérivé de cette dernière, conjugué(e) ou prévu(e) pour être conjugué(e) à un agent choisi parmi des radioisotopes, des agents paramagnétiques et des agents cytotoxiques et
d) un agent biotinylé pouvant rester dans le plasma pendant un certain laps de temps et pouvant se lier à l'avidine en circulation non fixée aux cellules tumorales et donc éliminer cette dernière, un tel agent étant choisi dans un groupe de protéines sériques comme l'albumine biotinylée, la transferrine biotinylée et les IgG biotinylées.

2. Ensemble selon la revendication 1, caractérisé en ce que la quantité totale du composant b) varie de 5 à 100 mg et est fournie sous une forme appropriée pour être administrée par voie intraveineuse en deux étapes successives.

3. Ensemble selon la revendication 1 ou 2, caractérisé en ce que la quantité mesurée du composant a) est comprise entre 0,5 et 10 mg.

4. Ensemble selon l'une quelconque des revendications précédentes, caractérisé en ce que la quantité mesurée du composant b) constitue un excédent molaire compris entre 10 et 100 fois la quantité mesurée du composant a).

5. Ensemble selon l'une quelconque des revendications précédentes, caractérisé en ce que le composant c) est radiomarqué par un radioisotope choisi dans un des groupes suivants: a) Fe-52; Mn-52m, Co-55, Cu-64, Ga-67, Ga-68, Tc-99m, In-111, I-123, I-125, I-131; b) P-32, Sc-47, Cu-67, Y-90, Pd-109, Ag-111, I-131, Pm-149, Re-186, Re-188, At-211, Pb-212, Bi-212.

6. Ensemble selon l'une quelconque des revendications 1 à 4, caractérisé en ce que le composant c) est conjugué à un agent paramagnétique choisi parmi le gadolinium, le fer et le manganèse.

7. Ensemble selon l'une quelconque des revendications 1 à 4, caractérisé en ce que le composant c) est conjugué à un agent cytotoxique choisi parmi le ricin, l'adriamycine et le platine-cis.

8. Ensemble selon l'une quelconque des revendications 1 à 4, dans lequel le composant c) est constitué par des liposomes biotinylés comprenant un agent chimiothérapeutique.

9. Ensemble selon l'une quelconque des revendications 1 ou 3-8, caractérisé en ce que la quantité mesurée du composant b) varie de 0,1 à 5 mg.

10. Ensemble selon la revendication 1 ou 2, caractérisé en ce que le composant b) est fourni en deux formes posologiques séparées :
i) 1/5 de la dose totale dans 3 ml de solution physiologique; et
ii) 4/5 de la dose dans 100 ml de solution physiologique.
